# FASCICULE DE BREVET EUROPEEN

(11) **EP 0 694 032 B1**
(45) Date de publication et mention de la délivrance du brevet: **26.07.2000**
(21) Numéro de dépôt: 94913644.4
(22) Date de dépôt: 14.04.1994
(51) Int. Cl.: C07D 205/08, C07D 409/04, C07D 417/04

(54) **PROCEDE DE PREPARATION DE BETA-LACTAMES**
VERFAHREN ZUR HERSTELLUNG VON BETA-LACTAMEN
METHOD FOR PREPARING BETA-LACTAMS

(30) Priorité: 16.04.1993 FR 9304495
(43) Date de publication de la demande: 31.01.1996
(73) Titulaire: Aventis Pharma S.A., 92160 Antony (FR)
(72) Inventeur: BOURZAT, Jean-Dominique, F-94300 Vincennes (FR); COMMER ON, Alain, F-94400 Vitry-sur-Seine (FR)
(74) Mandataire: Le Pennec, Magali
(86) Numéro de dépôt international: FR9400416
(87) Numéro de publication internationale: WO9424103

(56) Documents cités:
- EP-A- 0 400 971
- EP-A- 0 525 589
- JOURNAL OF ORGANIC CHEMISTRY vol. 58, no. 5 , 26 Février 1993 , EASTON US pages 1068 - 1075 R. BRIEVA 'Chemoenzymatic synthesis of the C-13 side chain of taxol: optically-active 3-hydroxy-4-phenyl beta-lactam derivatives.'
- CHEMICAL ABSTRACTS, vol. 118, no. 9, 1 Mars 1993, Columbus, Ohio, US; abstract no. 80677u, GUO,MAOJUN 'Studies on monocyclic beta-lactams. Synthesis of 3-hydroxy-4-substituted-2-azetidinones.' & GAODENG XUEXIAO HUAXUE XUEBAO no. 7 , 1992 pages 919 - 922
- TETRAHEDRON, (INCL. TETRAHEDRON REPORTS) vol. 48, no. 34 , 1992 , OXFORD GB pages 6985 - 7012 IWAO OJIMA 'New and efficient approaches to the semisynthesis of taxol and its C-13 side chain analogs by means of beta-lactam synthon method.' cité dans la demande

## Description

La présente invention concerne un nouveau procédé de préparation de β-lactames de formule générale : 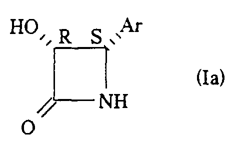 qui sont particulièrement utiles pour préparer des taxoïdes tels que le Taxotère ou le taxol.

Dans la formule générale (Ia), Ar représente un radical aryle.

Plus particulièrement, Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles, ou bien Ar représente un radical hétérocyclique aromatique ayant 5 chaînons et contenant un ou plusieurs atomes, identiques ou différents, choisis parmi les atomes d'azote, d'oxygène ou de soufre, éventuellement substitué par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

Plus particulièrement, Ar représente un radical phényle, thiényle-2 ou -3 ou furyle-2 ou -3 éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle.

Plus particulièrement encore, Ar représente un radical phényle éventuellement substitué par un atome de chlore ou de fluor, ou par un radical alcoyle (méthyle), alcoxy (méthoxy), dialcoylamino (diméthylamino), acylamino (acétylamino) ou alcoxycarbonylamino (tert-butoxycarbonylamino) ou thiényle-2 ou -3 ou furyle-2 ou -3.

Dans la demande de brevet européen EP 400 971 est décrite la préparation d'un β-lactame de formule générale (Ia) dans lequel la fonction hydroxy est protégée, par exemple, par un radical éthoxy-1 éthyle par condensation d'un chlorure d'acyloxyacétyle avec une N-benzylidène p.méthoxyaniline, suivie de l'élimination du radical p.méthoxyphényle et du remplacement du radical acyloxy par un groupement protecteur de la fonction hydroxy tel que le radical éthoxy-1 éthyle. Ce procédé conduit à la formation d'un produit racémique dont la résolution est nécessaire pour obtenir l'isomère 3R,4S qui est utile pour préparer les taxoïdes thérapeutiquement actifs.

Les hydroxy-3 aryl-4 azétidinones-2 ayant une pureté énantiomérique élevée peuvent être obtenues selon le procédé décrit par Ojima et coll., J. Org. Chem., 56, 1681-1683 (1991) qui met en oeuvre des (silyloxy) acétates chiraux qui ne sont pas commercialement accessibles et qui nécessitent pour leur préparation une résolution enzymatique.

Dans la demande de brevet européen EP 525 589 est décrite la préparation d'une β-lactame de formule générale (Ia) dans laquelle la fonction hydroxy est éventuellement estérifiée qui consiste à faire réagir une arylimine dérivée de la L-thréonine avec un halogénure d'acétyle, à séparer le diastéréoisomère désiré puis à éliminer l'auxiliaire chiral dérivé de la L-thréonine. Généralement, l'imine est obtenue in situ en faisant réagir le benzaldéhyde avec une L-thréonine dont la fonction hydroxy est protégée. Le mélange des diastéréoisomères, généralement dans le rapport 10/1 en faveur de l'énantiomère 3R,4S, est séparé par des méthodes habituelles telles que la cristallisation ou la chromatographie. La séparation peut être effectuée soit après la formation du cycle azétidine soit après l'élimination des groupements protecteurs. Ce procédé nécessite la mise en oeuvre d'un nombre important d'étapes.

Il a maintenant été trouvé, et c'est ce qui fait l'objet de la présente invention, que le produit de formule générale (Ia) peut être préparé stéréosélectivement par un procédé comportant un petit nombre d'étapes à partir de matières premières facilement et économiquement accessibles.

Selon la présente invention, les lactames de formule générale (Ia) peuvent être préparés à partir d'un mélange des diastéréoisomères des produits de formule générale : 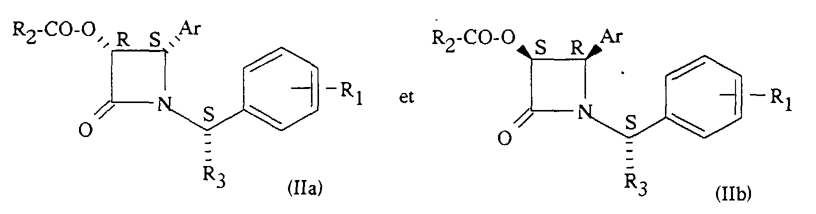 qui est obtenu par cyclo-addition d'un halogénure d'acide de formule générale : 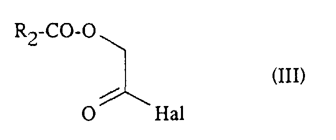 sur une imine chirale de formule générale :

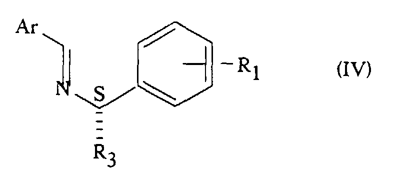

Dans les formules générales (IIa), (IIb), (III) et (IV),
Ar est défini comme précédemment,
R₁ représente un ou plusieurs substituants dont un doit être en position ortho ou para, identiques ou différents, choisis parmi les radicaux alcoxy contenant 1 à 4 atomes de carbone tel que le radical méthoxy, alcoylthio contenant 1 à 4 atomes de carbone tel que le radical méthylthio, ou dialcoylamino dont chaque partie alcoyle contient 1 à 4 atomes de carbone tel que le radical diméthylamino, R₂ représente un radical alcoyle contenant 1 à 4 atomes de carbone éventuellement substitué,
R₃ représente un radical alcoyle contenant 1 à 4 atomes de carbone, et
Hal représente un atome d'halogène tel qu'un atome de chlore ou de brome.

Selon l'invention, le β-lactame de formule générale (Ia) sous forme 3R,4S est issu du produit de formule générale (IIa) après remplacement de l'inducteur curai par un atome d'hydrogène pour obtenir un produit de formule générale : 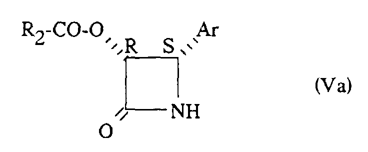 dans laquelle Ar et R₂ sont définis comme précédemment, qui est saponifié pour obtenir le produit de formule générale (Ia).

Le remplacement de l'inducteur chiral du produit de formule générale (IIa) par un atome d'hydrogène s'effectue généralement par hydrolyse en présence de nitrate de cérium et d'ammonium ou de dichloro-2,3 dicyano-5,6 p-benzoquinone, ou d'acétate mercurique, ou de bis(trifluoroacétoxy)iodobenzène en opérant dans l'eau ou dans un milieu hydroorganique à une température comprise entre 0 et 50°C, de préférence voisine de 20°C ou par oxydation électrochimique. Comme solvant organique, on utilise de préférence un nitrile tel que l'acétonitrile ou un ester tel que l'acétate de méthyle ou l'acétate d'éthyle.

La saponification de l'ester de formule générale (Va) est effectuée en milieu basique. De préférence, on utilise l'ammoniac en solution dans un alcool aliphatique tel que le méthanol ou l'éthanol.

Selon une mise en oeuvre de l'invention, on effectue le remplacement du groupement inducteur chiral sur le mélange des produits de formules générales (IIa) et (IIb) dans les conditions décrites précédemment, pour obtenir le mélange des produits de formules générales : 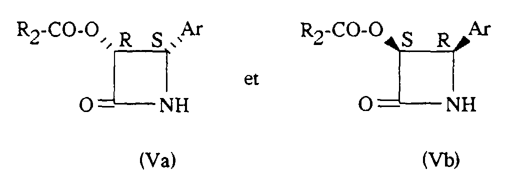 dans lequel Ar et R₂ sont définis comme précédemment, qui est saponifié dans les conditions décrites précédemment pour obtenir le mélange des produits de formules générales : 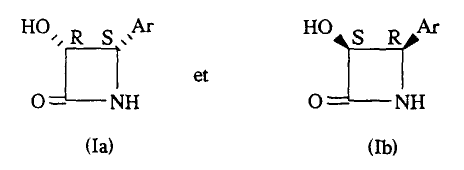 dont on sépare le produit de formule générale (Ia) par cristallisation ou par chromatographie sur phase chirale.

La séparation des produits de formule générale (Ia) et (Ib) s'effectue par cristallisation sélective dans un solvant organique approprié. Comme solvant organique, on utilise plus particulièrement un nitrile tel que l'acétonitrile ou un ester tel que l'acétate d'éthyle.
Le produit de formule générale (IIa) ou le mélange des produits de formule générale (IIa) et (IIb) est obtenu par action d'un halogénure d'acide de formule générale : 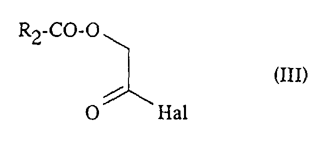 dans laquelle R₂ est défini comme précédemment et Hal représente un atome d'halogène, sur une imine chirale de formule générale : 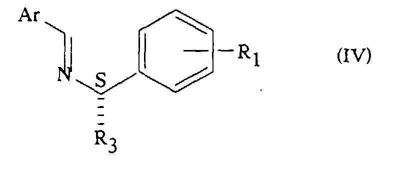 dans laquelle Ar, R₁, R₂ sont définis comme précédemment.

Selon un autre mode de mise en oeuvre du procédé selon l'invention, on effectue d'abord la saponification du mélange des produits de formule générale (IIa) et (IIb) pour obtenir le mélange des produits de formules générales : 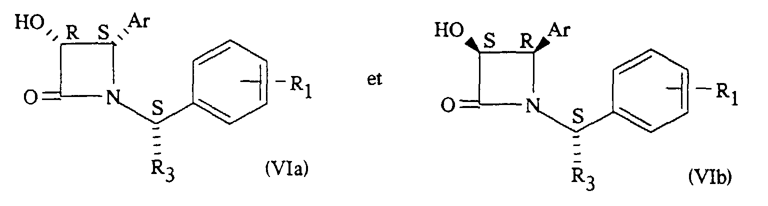 dont on sépare le constituant (VIa) qui est estérifié pour obtenir le produit de formule générale (IIa) dont on remplace l'inducteur chiral dans les conditions décrites précédemment pour obtenir le produit de formule générale (Va) qui est saponifié en produit de formule générale (Ia) dans les conditions décrites précédemment,
étant entendu que le mélange des produits de formule générale (IIa) et (IIb) est obtenu par action d'un halogénure d'acide de formule générale : 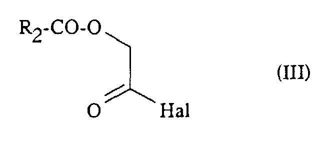 dans laquelle R₂ est défini comme précédemment et Hal représente un atome d'halogène sur une imine chirale de formule générale : 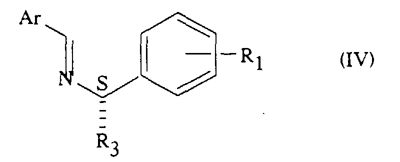 dans laquelle Ar, R₁ et R₃ sont définis comme précédemment.

La saponification du mélange des produits de formules générales (IIa) et (IIb) est effectuée en milieu basique. De préférence on utilise l'ammoniac dans un alcool aliphatique tel que le méthanol ou l'éthanol.

La séparation des produits de formules générales (VIa) et (VIb) est effectuée selon les méthodes habituelles telles que la cristallisation ou la chromatographie. De préférence, on effectue une cristallisation sélective dans un solvant organique approprié tel qu'un nitrile comme l'acétonitrile ou un ester comme l'acétate d'éthyle.

L'estérification du produit de formule générale (VIa) en produit de formule générale (IIa) est effectuée selon les méthodes habituelles par action d'un acide de formule générale :

R₂-CO-OH (VII)

dans laquelle R₂ est défini comme précédemment, ou d'un dérivé de cet acide tel qu'un halogénure, l'anhydride ou un anhydride mixte sur le produit de formule générale (VIa). De préférence, on utilise un halogénure d'acide en présence d'un agent basique tel qu'une base minérale comme le bicarbonate de sodium ou organique telle qu'une amine tertiaire comme la triéthylamine ou la pyridine.

Le mélange des produits de formules générale (IIa) et (IIb) est obtenu par action du produit de formule générale (III) sur le produit de formule générale (IV) en opérant généralement à une température comprise entre -20°C et 50°C, de préférence au voisinage de 0°C en présence d'une base choisie parmi les amines tertiaires (triéthylamine, N-méthylmorpholine, diisopropyléthylamine) ou la pyridine dans un solvant organique choisi parmi les hydrocarbures aliphatiques éventuellement halogénés tels que le chlorure de méthylène ou le chloroforme et les hydrocarbures aromatiques tels que le benzène, le toluène ou les xylènes.

Le produit de formule générale (IV) peut être obtenu dans les conditions décrites par M. Furukawa et coll., Chem. Pharm. Bull., 25, 181-184 (1977).

Les produits de formule générale (Ia) obtenus selon le procédé de la présente invention sont particulièrement utiles pour préparer des taxoïdes tels que le Taxotère, le taxol ou leurs analogues en opérant par exemple dans les conditions décrites dans EP 400 971 ou, après ouverture en milieu acide, selon I. Ojima et coll., J. Org. Chem., 56, 1681-1683 (1991), en opérant dans les conditions décrites dans les brevets européens EP 0 336 840 et EP 0 336 841.

Les exemples suivants illustrent la présente invention.

### EXEMPLE 1

A une solution de 1,9 g d'un mélange des deux épimères d'acétoxy-3 phényl-4 azétidinone-2, forme (3R,4S) et forme (3S,4R), dans 20 cm3 de méthanol on injecte, sous agitation, un courant gazeux d'ammoniac anhydre à une température voisine de 20°C pendant 1 heure. Le milieu réactionnel est concentré à sec sous pression réduite (2,7 kPa) à 40°C. Le solide résiduel est recristallisé dans 10 cm3 d'acétate d'éthyle pour donner 1,2 g de cristaux blancs dont le pouvoir rotatoire est : [α]²⁰_{D}= +117° (c = 0,52; méthanol). Ces cristaux sont recristallisés trois autres fois dans l'acétonitrile jusqu'à pouvoir rotatoire constant. On obtient ainsi 0,40 g d'hydroxy-3 phényl-4 azétidinone-2-(3R,4S) sous forme de cristaux blancs fondant à 191°C et dont les caractéristiques physiques sont en tous points identiques à celles du produit décrit par Iwao Ojima et al., Tetrahedron., 1992, **48**(34), 6985-7012.
- [α]²⁰_{D}=+182° (c = 0,65 ; méthanol)
- spectre de R.M.N.: (200 MHz ; DMSO d6 ; δ en ppm).

4,75 (d, J = 5 Hz, 1H : -CHC₆H₅) ; 4,99 (t large, J = 5 Hz, 1H ; -CHOH) ; 5,88 (d, J = 5 Hz, 1H : -OH) ; 7,25 à 7,45 (mt, 5H: -C₆H₅) ; 8,52 (s large, 1H : =NH) .

Le mélange des deux épimères d'acétoxy-3 phényl-4 azétidinone-2, forme (3R,4S) et forme (3S,4R), peut être préparé de la manière suivante :

A une solution de 5,1 g d'un mélange en proportion molaire 75/25 des deux diastéréolsomères d'acétoxy-3 [(méthoxy-4 phényl)-1-(S)]éthyl-1 phényl-4 azétidinone-2, respectivement forme (3R,4S) et forme (3S,4R), dans 165 cm3 d'acétonitrile on ajoute, goutte à goutte en 45 minutes, sous agitation et à uné température voisine de 0°C, une solution de 27,5 g de nitrate d'ammonium et de cérium dans 250 cm3 d'eau distillée. La solution obtenue est agitée pendant 30 minutes à 0°C puis 1 heure à une température voisine de 20°C et additionnée d'hydrogénocarbonate de sodium jusqu'à saturation. Le milieu réactionnel est extrait par 3 fois 150 cm3 d'acétate d'éthyle. Les phases organiques sont réunies, lavées par 2 fois 25 cm3 d'eau distillée, séchées sur sulfate de magnésium, filtrées puis concentrées à sec sous pression réduite (2,7 kPa) à 40°C. On obtient 5,1 g de cristaux blancs que l'on purifie par chromatographie sur 190 g de silice (0,063-0,2 mm) contenus dans une colonne de 4 cm de diamètre (éluant: dichlorométhane) en recueillant des fractions de 20 cm3. Les fractions ne contenant que le produit cherché sont réunies et concentrées à sec sous pression réduite (2,7 kPa) à 40°C. On obtient ainsi 2,8 g de cristaux blancs que l'on recristallise dans 20 cm3 d'acétate d'éthyle pour donner 2,2 g d'un mélange des deux épimères d'acétoxy-3 phényl-4 azétidinone-2, forme (3R,4S) et forme (3S,4R), sous forme de cristaux blancs fondant à 170°C et dont les caractéristiques physiques sont les suivantes :
- [α]²⁰_{D}= -8,2° (c = 0,78; méthanol)
- spectre de R.M.N.: (300 MHz ; CDCl₃ ; δ en ppm).

1,68 (s, 3H : -OCOCH₃) ; 5,04 (d, J = 5 Hz, 1H : -CHOCOCH₃) ; 5,88 (dd, J = 5 et 2,5 Hz, 1H : -CHC₆H₅) ; 6,63 (mf, 1H : =NH) ; 7,25 à 7,45 (mt, 5H : -C₆H₅).

Le mélange en proportion molaire 75/25 des deux diastéréoisomères d'acétoxy-3 [(méthoxy-4 phényl)-1-(S)]éthyl-1 phényl-4 azétidinone-2, respectivement forme (3R,4S) et forme (3S,4R), peut être préparé de la manière suivante :

A une solution de 13,2 g de N-benzylidène-[(méthoxy-4 phényl)-1 éthylamine]-(S) dans 100 cm3 de chloroforme on ajoute, sous agitation et à une température voisine de 20°C, 10,3 cm3 de triéthylamine puis on refroidit le mélange réactionnel jusqu'à une température voisine de -20°C et additionne goutte à goutte, en 75 minutes et en se maintenant à cette température, une solution de 4 cm3 de chlorure d'acétoxy-2 acétyle dans 50 cm3 de chloroforme. La solution obtenue est agitée pendant 16 heures à une température voisine de 20°C puis additionnée de 40 cm3 d'eau distillée et de 200 cm3 de dichlorométhane. La phase organique est séparée par décantation, lavée par 20 cm3 d'eau distillée puis successivement, par 50 cm3 d'une solution aqueuse 1N d'acide chlorhydrique, par 20 cm3 d'eau distillée, par 30 cm3 d'une solution aqueuse saturée d'hydrogénocarbonate de sodium et par 2 fois 10 cm3 d'eau distillée, séchée sur sulfate de magnésium, filtrée puis concentrée à sec sous pression réduite (2,7 kPa) à 40°C. On obtient ainsi 16 g d'une huile brune que l'on purifie par chromatographie sur 100 g de silice (0,063-0,2 mm) contenus dans une colonne de 3 cm de diamètre (éluant: dichlorométhane) recueillant des fractions de 20 cm3. Les fractions ne contenant que le produit cherché sont réunies et concentrées à sec sous pression réduite (2,7 kPa) à 40°C. On obtient ainsi 11,8 g de cristaux blancs que l'on recristallise dans 15 cm3 d'oxyde de diisopropyle pour donner 4,4 g d'un mélange en proportion molaire 75/25 des deux diastéréoisomères d'acétoxy-3 [(méthoxy-4 phényl)-1-(S)]éthyl-1 phényl-4 azétidinone-2, respectivement forme (3R,4S) et forme (3S,4R), sous forme de cristaux blancs fondant à 70°C.

La N-benzylidène-[(méthoxy-4 phényl)-1 éthylaminel]-(S) peut être préparée de la manière suivante :

A une solution de 5,6 g de benzaldéhyde dans 25 cm3 de dichlorométhane on ajoute, sous agitation et à une température voisine de 20°C, 8,5 g de (méthoxy-4 phényl)-1 éthylamine-(S) et 5 g de tamis moléculaire 4 Å. Le mélange réactionnel est agité pendant 16 heures à une température voisine de 20°C puis filtré sur verre fritté garni de célite. Le verre fritté est lavé par 3 fois 20 cm3 de dichlorométhane et les filtrats sont réunis puis concentrés à sec sous pression réduite (2,7 kPa) à une température voisine de 40°C. On obtient ainsi 13,4 g de N-benzylidène- [(méthoxy-4 phényl)-1 éthylamine]-(S) sous forme d'une huile brun clair.
- [α]²⁰_{D}= +13,4° (c = 0,70 ; méthanol)

La (méthoxy-4 phényl)-1 éthylamine-(S) peut être préparé selon la méthode décrite par H.O. Bernhardt et al., Helv. Chim. Acta., 1973, **56**(4), 1266-1303.

### EXEMPLE 2

A une solution de 2,0 g d'acétoxy-3 (thiényl-3)-4 azétidinone-2-(3R,4S), dans 100 cm3 de méthanol on injecte, sous agitation, un courant gazeux d'ammoniac anhydre à une température voisine de 20°C pendant 1 heure. Le milieu réactionnel est concentré à sec sous pression réduite (2,7 kPa) à 40°C. On obtient ainsi 2,0 g de cristaux blancs que l'on recristallise dans 8 cm3 d'acétate d'éthyle pour donner 1,3 g d'hydroxy-3 (thiényl-3)-4 azétidinone-2-(3R,4S) sous forme de cristaux blancs fondant à 130°C et dont les caractéristiques physiques sont les suivantes :
- [α]²⁰_{D}= +119° (c = 0,64 ; méthanol)
- spectre de R.M.N. : (300 MHz; DMSO d6 ; δ en ppm).

4,75 [(d, J = 4,5 Hz, 1H : -CH-(thiényl-3)] ; 4,92 (dd, J = 7 et 4,5 Hz, 1H : -CHOH) ; 5,92 (d, J = 7 Hz, 1H: -OH) ; 7,04 (dd, J = 5 et 1,5 Hz, 1H: -H en 4 du thiényl-3) ; 7,35 (dd, J = 3 et 1,5 Hz, 1H : -H en 2 du thiényl-3) ; 7,51 (dd, J = 5 et 3 Hz, 1H : -H en 5 du thiényl-3) ; 8,50 (s, 1H: =NH).

L'acétoxy-3 (thiényl-3)-4 azétidinone-2-(3R,4S) peut être préparé de la manière suivante :

A une solution de 5,1 g d'acétoxy-3 [(méthoxy-4 phényl)-1-(S)]éthyl-1 (thiényl-3)-4 azétidinone-2-(3R,4S) dans 150 cm3 d'acétonitrile on ajoute, goutte à goutte en 45 minutes, sous agitation et à une température voisine de 0°C, une solution de 27 g de nitrate d'ammonium et de cérium dans 225 cm3 d'eau distillée. La solution obtenue est agitée pendant 30 minutes à 0°C puis 1 heure à une température voisine de 20°C et additionnée d'hydrogénocarbonate de sodium jusqu'à saturation. Le milieu réactionnel est extrait par 3 fois 200 cm3 d'acétate d'éthyle. Les phases organiques sont réunies, lavées par 4 fois 15 cm3 d'eau distillée, séchées sur sulfate de magnésium, filtrées puis concentrées à sec sous pression réduite (2,7 kPa) à 40°C. On obtient ainsi 5 g de cristaux blancs que l'on purifie par chromatographie sur 190 g de silice (0,063-0,2 mm) contenus dans une colonne de 3,5 cm de diamètre [éluant: dichlorométhane-méthanol (99,5-0,5 en volumes)] en recueillant des fractions de 20 cm³. Les fractions ne contenant que le produit cherché sont réunies et concentrées à sec sous pression réduite (2,7 kPa) à 40°C. On obtient ainsi 2,1 g d'acétoxy-3 (thiényl-3)-4 azétidinone-2-(3R,4S) sous forme de cristaux blancs fondant à 168°C et dont les caractéristiques physiques sont les suivantes :
- spectre de R.M.N. : (200 MHz ; CDCl₃; δ en ppm).

1,80 (s, 3H: -OCOCH₃) ; 5,12 (d, J = 5 Hz, 1H: -CHOCOCH₃) ; 5,85 [dd, J = 5 et 2,5 Hz, 1H: -CH-(thiényl-3)] ; 6,41 (mf, 1H : =NH) ; 7,04 (dd, J = 5 et 1 Hz, 1H : -H en 4 du thiényl-3) ; 7,27 (mi masqué par la bande de solvant résiduel : -H en 2 du thiényl-3) ; 7,35 (dd, J = 5 et 3 Hz, 1H : -H en 5 du thiényl-3).

L'acétoxy-3 [(méthoxy-4 phényl)-1-(S)]éthyl-1 (thiényl-3)-4 azétidinone-2-(3R,4S) peut être préparé de la manière suivante :

A une solution de 5,1 g d'hydroxy-3 [(méthoxy-4 phényl)-1-(S)]éthyl-1 (thiényl-3)-4 azétidinone-2-(3R,4S) dans 75 cm3 de dichlorométhane on ajoute 1,5 cm3 de pyridine puis, goutte à goutte en 15 minutes sous agitation et à une température voisine de 20°C, une solution de 1,36 cm3 de chlorure d'acétyle dans 10 cm3 de dichlorométhane. La solution obtenue est agitée pendant 2,5 heures à une température voisine de 20°C puis additionnée de 0,75 cm3 de pyridine, de 0,45 cm3 de chlorure d'acétyle et maintenue sous agitation à une température voisine de 20°C pendant 1 heure. Le milieu réactionnel est ensuite additionné de 25 cm3 d'eau distillée et agité pendant 5 minutes. La phase aqueuse est séparée par décantation puis extraite par 50 cm3 de dichlorométhane. Les phases organiques sont réunies, lavées par 2 fois 20 cm3 d'eau distillée, séchées sur sulfate de magnésium, filtrées puis concentrées à sec sous pression réduite (2,7 kPa) à 40°C. On obtient ainsi 5,9 g d'un solide blanc que l'on purifie par chromatographie sur 110 g de silice (0,063-0,2 mm) contenus dans une colonne de 3 cm de diamètre [éluant: dichlorométhane-méthanol (99-1 en volumes)] en recueillant des fractions de 20 cm3. Les fractions ne contenant que le produit cherché sont réunies et concentrées à sec sous pression réduite (2,7 kPa) à 40°C.

On obtient ainsi 5,6 g d'acétoxy-3 (thiényl-3)-4 azétidinone-2-(3R,4S) sous forme de cristaux blancs fondant à 60°C et dont les caractéristiques physiques sont les suivantes :
- spectre de R.M.N. : (200 MHz ; CDCl3 ; δ en ppm).

1,32 (d, J = 7 Hz, 3H : -CHCH₃) ; 1,71 (s, 3H : -OCOCH₃) ; 3,75 (s, 3H: -OCH₃) ; 4,62 et 5,51 [(2d, J = 5 Hz, 1H chacun : -CHOCOCH₃ et -CH-(thiényl-3)] ; 4,87 (q, J = 7 Hz, 1H : -CHCH₃) ; 6,73 [d, J = 8,5 Hz, 2H : -C₆H₄OCH₃(-H 3 et -H 5)] ; 6,89 (dd, J = 5 et 1,5 Hz, 1H : -H en 4 du thiényl-3) ; 7,02 [(mt, 3H : -C₆H₄OCH₃(-H 2 et -H 6) et -H en 2 du thiényl-3)] ; 7,16 (dd, J = 5 et 3 Hz, 1H : -H en 5 du thiényl-3).

L'hydroxy-3 [(méthoxy-4 phényl)-1-(S)]éthyl-1 (thiényl-3)-4 azétidinone-2-(3R,4S) peut être préparé de la manière suivante :

A une solution de 10,5 g d'un mélange en proportion molaire 75/25 des deux diastéréoisomères d'acétoxy-3 [(méthoxy-4 phényl)-1-(S)]éthyl-1 (thiényl-3)-4 azétidinone-2, respectivement forme (3R,4S) et forme (3S,4R), dans 150 cm3 de méthanol on injecte, sous agitation, un courant gazeux d'ammoniac anhydre à une température voisine de 20°C pendant 2,5 heures. Le milieu réactionnel est ensuite concentré à sec sous pression réduite (2,7 kPa) à 40°C. Le solide résiduel est recristallisé une première fois dans 25 cm3 d'acétate d'éthyle puis une seconde fois dans 18 cm3 d'acétonitrile. On obtient ainsi 5,2 g d'hydroxy-3 [(méthoxy-4 phényl)-1-(S)]éthyl-1 (thiényl-3)-4 azétidinone-2-(3R,4S) sous forme de cristaux blancs fondant à 150°C et dont les caractéristiques sont les suivantes:
- spectre de R.M.N. : (200 MHz ; CDCl3 ; δ en ppm).

1,38 (d, J = 7,5 Hz, 3H : -CHCH₃) ; 3,60 (d, J = 7,5 Hz, 1H : -OH) ; 3,81 (s, 3H : -OCH₃) ; 4,63 [d, J = 5 Hz, 1H : -CH-(thiényl-3)] ; 4,89 (dd, J = 7,5 et 5 Hz, 1H : -CHOH) ; 4,97 (q, J = 7,5 Hz, 1H : -CHCH₃) ; 6,85 [d, J = 8 Hz , 2H : -C₆H₄OCH₃(-H 3 et -H 5)] ; 7,11 (dd, J = 5 et 1,5 Hz, 1H : -H en 4 du thiényl-3) ; 7,15 (d, J = 8 Hz, 2H : -C₆H₄OCH₃(-H 2 et -H6)] ; 7,22 (dd, J = 3 et 1,5 Hz, 1H : -H en 2 du thiényl-3) ; 7,37 (dd, J = 5 et 3 Hz, 1H : -H en 5 du thiényl-3).

Le mélange en proportion molaire 75/25 des deux diastéréoisomères d'acétoxy-3 [(méthoxy-4 phényl)-1-(S)]éthyl-1 (thiényl-3)-4 azétidinone-2, respectivement forme (3R,4S) et forme (3S,4R), peut être préparé de la manière suivante :

A une solution de 9,2 g de N-(thiényl-3)méthylidène-[(méthoxy-4) phényl-1 éthylamine]-(S) dans 75 cm3 de chloroforme on ajoute, sous agitation et à une température voisine de 20°C, 10,5 cm3 de triéthylamine puis on refroidit le mélange réactionnel jusqu'à une température voisine de -20°C et ajoute goutte à goutte, en 75 minutes et en se maintenant à cette température, une solution de 4 cm3 de chlorure d'acétoxy-2 acétyle dans 40 cm3 de chloroforme. La solution obtenue est agitée pendant 16 heures à une température voisine de 20°C puis additionnée de 40 cm3 d'eau distillée et de 200 cm3 de dichlorométhane.

La phase organique est séparée par décantation, lavée par 40 cm3 d'eau distillée puis successivement, par 75 cm3 d'une solution aqueuse 1N d'acide chlorhydrique, par 25 cm3 d'eau distillée, par 50 cm3 d'une solution aqueuse saturée d'hydrogénocarbonate de sodium et par 2 fois 25 cm3 d'eau distillée, séchée sur sulfate de magnésium, filtrée puis concentrée à sec sous pression réduite (2,7 kPa) à 40°C. On obtient ainsi 16,5 g d'une huile brune que l'on purifie par chromatographie sur 170 g de silice (0,063-0,2 mm) contenus dans une colonne de 3,5 cm de diamètre [éluant : dichlorométhane-méthanol (99,5-0,5 en volumes)] en recueillant des fractions de 20 cm3. Les fractions ne contenant que le produit cherché sont réunies et concentrées à sec sous pression réduite (2,7 kPa) à 40°C. On obtient ainsi 10,6 g d'un mélange en proportion molaire 75/25 des deux diastéréoisomères d'acétoxy-3 [(méthoxy-4 phényl)-1-(S)]éthyl-1 (thiényl-3)-4 azétidinone-2, respectivement forme (3R,4S) et forme (3S,4R), sous forme d'une huile épaisse jaune.

La N-(thiényl-3) méthylidène-[(méthoxy-4)phényl-1 éthylamine]-(S) peut être préparée de la manière suivante :

A une solution de 3,5 g de thiophènecarbaldédyde-3 dans 20 cm3 de dichlorométhane on ajoute, sous agitation et à une température voisine de 20°C, 5,8 g de (méthoxy-4 phényl)-1 éthylamine-(S) et 4 g de tamis moléculaire 4 Å. Le mélange réactionnel est agité pendant 16 heures à une température voisine de 20°C puis filtré sur verre fritté garni de célite. Le verre fritté est lavé par 3 fois 15 cm3 de dichlorométhane et les filtrats sont réunis puis concentrés à sec sous pression réduite (2,7 kPa) à une température voisine de 40°C. On obtient ainsi 9,25 g de N-(thiényl-3)méthylidène-[(méthoxy-4 phényl)-1 éthylamine]-(S) sous forme de cristaux ocre fondant à 60°C.

### EXEMPLE 3

A une solution de 3,88 g d'acétoxy-3 (thiényl-2)-4 azétidinone-2-(3R,4R) dans 60 cm3 de méthanol, on injecte, sous agitation, un courant gazeux d'ammoniac anhydre à une température voisine de 5°C pendant une heure. Le milieu réactionnel est concentré à sec sous pression réduite (2,7 kPa) à 40°C. Le solide résiduel est purifié par chromatographie sur 180 g de silice (0,063-0,2 mm) contenus dans une colonne de 4 cm de diamètre [éluant : dichlorométhane-méthanol (95-5 en volumes)] en recueillant des fractions de 10 cm3. Les fractions ne contenant que le produit cherché sont réunies et concentrées à sec sous pression réduite (2,7 kPa) à 40°C. On obtient ainsi 2,24 g d'hydroxy-3 (thiényl-2)-4 azétidinone-2-(3R,4R) sous forme de cristaux blancs fondant à 174°C et dont les caractéristiques physiques sont les suivantes :
- [α]²⁰_{D} = +121 (c = 0,53 ; méthanol).
- spectre de R.M.N. : [200 MHz ; DMSO d₆ ; δ en ppm] : 4,96 [ab limite, 2H : -CH-(thiényl-2) et -CHOH] ; 6,05 (mf étalé, 1H : -OH); de 6,95 à 7,10 (mt 2H : -H en 4 et -H en 3 du thiényl-2) ; 7,47 (mt, 1H : -H en 5 du thiényl-2) ; 8,64 (mf, 1H : -NH-).

L'acétoxy-3 (thiényl-2)-4 azétidinone-2-(3R,4R) peut être préparée de la manière suivante :

A une solution de 3,75 g d'acétoxy-3 [(diméthoxy-3,4 phényl)-1-(S)]éthyl-1 (thiényl-2)-4 azétidinone-2-(3R,4R) dans 110 cm3 d'acétonitrile, on ajoute, goutte à goutte en 75 minutes, sous agitation et à une température voisine de -5°C, une solution de 21,84 g de nitrate d'ammonium et de cérium dans 180 cm³ d'une solution aqueuse saturée de chlorure de sodium. A la fin de l'addition, on ajoute 100 m3 d'une solution aqueuse saturée d'hydrogénocarbonate de sodium. Le milieu réactionel est extrait par 3 fois 150 cm3 d'acétate d'éthyle. Les phases organiques sont réunies, lavées par 150 cm3 d'eau distillée puis séchées sur sulfate de magnésium, filtrées et concentrées à sec sous pression réduite (2,7 kPa) à 40°C. On obtient ainsi 4,1 g d'une meringue crème que l'on purifie par chromatographie sur 400 g de silice (0,063-0,2 mm) contenus dans une colonne de 3 cm de diamètre [éluant : dichlorométhaneméthanol (98-2 en volumes)] en recueillant des fractions de 15 cm3. Les fractions ne contenant que le produit cherché sont réunies et concentrées à sec sous pression réduite (2,7 kPa) à 40°C. On obtient ainsi 1,40 g d'acétoxy-3 (thiényl-2)-4 azétidinone-2-(3R,4R) sous forme de cristaux blancs fondant à 176°C et dont les caractéristiques physiques sont les suivantes :
- [α]²⁰_{D} = -63 (c = 0,49 ; méthanol).
- spectre de R.M.N. : [200 MHz ; DMSO d₆ ; δ en ppm] : 1,82 (s, 3H: -OCOCH₃) ; 5,28 (d, J = 4,5 Hz, 1H : -CHOCOCH₃) ; 5,83 [dd, J = 4,5 et 2,5 Hz, 1H : -CH-(thiényl-2)] ; de 7,95 à 7,10 (mt, 2H : -H en 4 et -H en 3 du thiényl-2) ; 7,60 (d large, J = 4,5 Hz, 1H : -H en 5 du thiényl-2) ; 9,00 (mf, 1H : -NH-).

L'acétoxy-3 [(diméthoxy-3,4 phényl)-1-(S)]éthyl-1 (thiényl-2)-4 azétidinone-2-(3R,4R) peut être préparée de la manière suivante:

A une solution de 45,95 g de N-(thiényl-2)méthylidène-[(diméthoxy-3,4 phényl)-1 éthylamine]-(S) dans 450 cm3 de chloroforme et 70 cm3 de triéthylamine, on ajoute, goutte à goutte en 3 heures, sous agitation et à une température voisine de -30°C, une solution de 22,1 cm3 de chlorure d'acétoxyacétyle dans 150 cm3 de chloroforme. L'addition terminée, le milieu réactionnel est réchauffé jusqu'à une température voisine de 20°C et maintenu sous agitation à cette température pendant 15 heures. On additionne ensuite 200 cm3 d'une solution aqueuse saturée de chlorure d'ammonium. La phase organique est décantée, lavée par 2 fois 200 cm3 d'eau distillée puis séchée sur sulfate de magnésium, filtrée et concentrée à sec sous pression réduite (2,7 kPa) à 40°C. On obtient 76,7 g de cristaux marrons qui sont recristallisés dans 250 cm3 d'éthanol absolu pour donner 25,4 g d'acétoxy-3 (diméthoxy-3,4 phényl)-1 (thiényl-2)-4 azétidinone-2-(3R,4R) sous forme de cristaux blancs fondant à 100°C et dont les caractéristiques physiques sont les suivantes :
- [α]²⁰_{D} = -8 (c = 0,50) ; méthanol)
- spectre de R.M.N. : (200 MHz ; CDCl₃ ; δ en ppm) : 1,45 (d, J = 7,5 Hz, 3H : -CHCH₃) ; 1,87 (s, 3H: -COCH₃) ; 3,82 et 3,89 (2s, 3H chacun: -OCH₃) ; 4,91 et 5,69 [2d, J = 4,5 Hz, 1H chacun : -CHOCOCH₃ et -CH-(thiényl-2)] ; 4,99 (q, J = 7,5 Hz, 1H : -CHCH₃) ; de 6,70 à 7,05 [mt, 5H: -C₆H₃(OCH₃)₂(-H 2, H 5, H 6), -H en 4 et -H en 3 du thiényl-2] ; 7,33 (dd, J = 4,5 et 2 Hz, 1H : -H en 5 du thiényl-2).

La N-(thiényl-2)méthylidène-[(diméthoxy-3,4 phényl)-1 éthylamine]-(S) peut être préparée de la manière suivante :

A une solution de 43,8 g de (diméthoxy-3,4 phényl)-1 éthylamine-(S) dans 400 cm3 de dichlorométhane, on ajoute sous agitation, à une température voisine de 20°C, 16,8 cm3 de thiophène-2 carboxaldéhyde et 35 g de tamis moléculaire 4Å. Le mélange réactionnel est agité pendant 16 heures à une température voisine de 20°C puis filtré sur verre fritté garni de célite. Le verre fritté est lavé par 3 fois 50 cm3 de dichlorométhane et les filtrats sont réunis puis concentrés à sec sous pression réduite (2,7 kPa) à 40°C. On obtient ainsi 45,95 g de N-(thiényl-2)méthylidène]-[(diméthoxy-3,4 phényl)-1 éthylamine)-(S) sous forme d'une huile incolore dont les caractéristiques sont les suivantes :
- [α]²⁰_{D} = -20 (c = 0,52; méthanol).
- spectre de R.M.N. : (200 MHz ; CDCl₃ ; δ en ppm) : 1,58 (d, J = 7 Hz, 3H : -CHCH₃) ; 3,86 et 3,90 (2s, 3H chacun: -OCH₃) ; 4,48 (q, J = 7 Hz, 1H : -CHCH₃) ; 6,84 (d, J = 8 Hz, 1H : -C₆H₃(OCH₃)₂(-H 5) ; 6,88 (dd, J = 8 et 1,5 Hz, 1H : -C₆H₃(OCH₃)₂(-H 6) ; 6,98 (d, J = 1,5 Hz, 1H : -C₆H₃(OCH₃)₂(-H2) ; 7,06 (dd, J = 4,5 ex 3 Hz, 1H : -H en 4 du thiényl-2) ; 7,29 (d large, J = 3 Hz, 1H : -H en 3 du thiényl-2) ; 7,39 (d large, J = 4,5 Hz, 1H : -H en 5 du thiényl-2) ; 8,41 (s, 1H : -CH=N-).

La (diméthoxy-3,4 phényl)-1 éthylamine-(S) peut être préparée selon la méthode décrite par V.M. Potatov et al., Vestn. Mosk. Univ., Ser. 2 : Khim. 1997, 18(4), 446 (CA: 88, 62074 c).

### EXEMPLE 4

En opérant comme dans l'exemple 2, mais à partir de 0,8 g d'acétoxy-3 (thiazolyl-4)-4 azétidinone-2-(3R,4S), on obtient 0,52 g d'hydroxy-3 (thiazolyl-4)-4 azétidinone-2-(3R,4S) sous forme de cristaux beige fondant à 190°C et dont les caractéristiques physiques sont les suivantes :
- [α]²⁰_{D}=+112 (c = 0,51 ; méthanol)
- spectre de R.M.N. : (200 MHz ; DMSO d₆ ; δ en ppm) : 4,91 [(d, J = 5 Hz, 1H : -CH-(thiazolyl-4)] ; 5,00 (dd, J = 8 et 5 Hz, 1H : -CHOH) ; 5,93 (d, J = 8 Hz, 1H : -OH) ; 7,50 (d, J = 2 Hz, 1H : -H en 5 du thiazolyl-4) ; 8,55 (s large, 1H : -NH-) ; 9,10 (d, J = 2 Hz, 1H : -H en 2 du thiazolyl-4).

En opérant comme dans l'exemple 2, à partir des matières premières convenables, sont préparés les intermédiaires suivants :
- acétoxy-3 (thiazolyl-4)-4 azétidinone-2-(3R,4S) sous forme de cristaux blancs fondant à 155°C et dont les caractéristiques physiques sont les suivantes :
- spectre de R.M.N. : (200 MHz ; CDCl₃ ; δ en ppm) : 1,85 (s, 3H : -OCOCH₃) ; 5,35 (d, J = 5 Hz, 1H: -CHOCOCH₃) ; 6,03 [(dd, J = 5 et 2,5 Hz, 1H: -CH-(thiazolyl-4)] ; 6,45 (d, J = 2,5 Hz, 1H : -NH-) ; 7,4 (d, J = 2Hz, 1H : -H en 5 du thiazolyl-4) ; 8,86 (d, J = 2Hz, 1H : -H en 2 du thiazolyl-4).
- acétoxy-3 [(méthoxy-4 phényl)-1-(S)]éthyl-1 (thiazolyl-4)-4 azétidinone-2-(3R,4S) sous forme de cristaux blancs fondant à 110°C et dont les caractéristiques physiques sont les suivantes
- spectre de R.M.N. : (300 MHz ; CDCl₃ ; δ en ppm) : 1,39 (d, J = 7 Hz, 3H : -CHCH₃) ; 1,80 (s, 3H: -OCOCH₃) ; 3,80 (s, 3H: -OCH₃) ; 4,97 (q, J = 7 Hz, 1H : -CHCH₃) ; 5,00 et 5,72 [2d, J = 5 Hz, 1H chacun : -CHOCOCH₃ et -CH-(thiazolyl-4)] ; 6,84 [d, J = 7,5 Hz, 2H : -C₆H₄OCH₃ (-H 3 et -H 5)] ; 7,13 [d, J = 7,5 Hz, 2H : -C₆H₄OCH₃(-H 2 et -H 6] ; 7,19 (d, J = 2 Hz, 1H : H en 5 du thiazolyl-4) ; 8,79 (d, J = 2 Hz 1H : H en 2 du thiazolyl-4).
- hydroxy-3 [(méthoxy-4 phényl)-1-(S)]éthyl-1 (thiazolyl-4)-4 azétidinone-2-(3R,4S) sous forme de cristaux blancs fondants à 90°C et dont les caractéristiques sont les suivantes :
- spectre de R.M.N. : (200 MHz ; CDCl₃ ; δ en ppm) : 1,25 (d, J = 7 Hz, 3H : -CHCH₃) ; 3,78 (s, 3H : -OCH₃) ; 4,53 (d, J = 11 Hz, 1H: -OH) ; 4,65 [(d, J = 5 Hz, 1H : -CH-(thiazolyl-4)] ; 4,93 (q, J = 7 Hz, 1H: -CHCH₃) ; 5,02 (dd, J = 11 et 5 Hz, 1H : -CHOH) ; 6,85 [(d, J = 7,5 Hz, 2H: -C₆H₄OCH₃(-H 3 et -H 5)] ; 7,13 [(d, J = 7,5 Hz, 2H : -C₆H₄OCH₃(-H 2 et -H 6)] ; 7,19 (d, J = 2 Hz, 1H : -H en 5 du thiazolyl-4) ; 8,85 (d, J =2 Hz, 1H : -H en 2 du thiazolyl-4).
- le mélange en proportion molaire (75/25) des deux diastéréoisomères d'acétoxy-3 [(méthoxy-4 phényl)-1-(S)]éthyl-1 (thiazolyl-4)-4 azétidinone-2, respectivement forme (3R,4S) et forme (3S,4R) sous forme d'une huile épaisse jaune.
- la N-(thiazolyl-4)méthylidène-[(méthoxy-4)phényl-1 éthylamine]-(S) sous forme d'une huile épaisse jaune.
- le thiazolecarbaldéhyde-4 préparé selon la méthode décrite par A. Dondoni et al., Synthesis., 1987, 998-1001

### EXEMPLE 5

En opérant comme dans l'exemple 2, mais à partir de 1,15 g d'acétoxy-3 (thiazolyl-5)-4 azétidinone-2-(3R,4S), on obtient 0,85 g d'hydroxy-3 (thiazolyl-5)-4 azétidinone-2-(3R,4R) sous forme de cristaux beige fondant à 180°C et dont les caractéristiques sont les suivantes :
- [α]²⁰_{D}= +74 (c = 0,45 ; méthanol)
- spectre de R.M.N. : (300 MHz; DMSO d₆ ; δ en ppm) : 5,00 (dd, J = 5 et 8 Hz, 1H : CHOH) ; 5,06 [dd, J = 5 Hz, 1H: -CH-(thiazolyl-5)] ; 6,23 (d, J = 8 Hz, 1H : -CH : -OH); 7,80 (s large, 1H : -H en 4 du thiazolyl-5) ; 8,69 (mt, 1H : -NH-) ; 9,02 (s, large, 1H : H en 2 du thiazolyl-5),

En opérant comme dans l'exemple 2, à partir de matières convenables, sont préparés les intermédiaires suivants :
- acétoxy-3 (thiazolyl-5)-4 azétidinone-2-(3R,4R) sous forme de cristaux blancs fondant à 140°C et dont les caractéristiques sont les suivantes :
- spectre de R.M.N. : (300 MHz; CDCl₃ ; δ en ppm) : 1,89 (s, 3H, -OCOCH₃) ; 5,36 (d, J = 4,5 Hz, 1H : -CHOCOCH₃) ; 5,90 [(mt, 1H : -CH-(thiazolyl-5)] ; 7,03 (mt, 1H: -NH-) ; 7,82 (s, 1H : -H en 4 du thiazolyl-5) ; 8,83 (s, 1H: -H en 2 du thiazolyl-5)
- acétoxy-3 (méthoxy-4 phényl)-1-(S)]éthyl-1 (thiazolyl-5)-4 azétidinone-2-(3R,4R)sous forme de cristaux blancs fondant à 90°C et dont les caractéristiques physiques sont les suivantes:
- spectre de R.M.N. : (300 MHz ; CDCl₃ ; δ en ppm) : 1,38 (d, J = 7 Hz, 3H : -CHCH₃) ; 1,90 (s, 3H: -OCOCH₃) ; 3,81 (s, 3H : -OCH₃) ; 4,97 (q, J = 7 Hz, 1H : -CHCH₃) ; 4,97 et 5,75 (2d, J = 5 Hz, 1H chacun : -CHOCOCH₃ et -CH-(thiazolyl-5)] ; 6,85 (d, J = 7,5 Hz, 2H : -C₆H₄OCH₃(-H 3 et -H 5)] ; 7,11 (d, J = 7,5 Hz, 2H : -C₆H₄OCH₃(-H 2 et -H 6) ; 7,71 (s, 1H : -H en 4 du thiazolyl-5) ; 8,83 (s, 1H : -H en 2 du thiazolyl-5).
- hydroxy-3 [(méthoxy-4 phényl)-1-(S)]éthyl-1(thiazolyl-5)-4 azétidinone-2-(3R,4R) : cristaux blancs fondants à 160 C et dont les caractéristiques sont les suivantes :
- spectre de R.M.N. : (300 MHz ; CDCl3 ; δ en ppm) : 1,39 (d, J = 7 Hz, 3H : -CHCH₃) ; 3,82 (s, 3H : -OCH₃) ; 4,85 et 5,00 [2d, J = 5 Hz, 1H chacun : -CH-(thiazolyl-5) et -CHOH] ; 4,95 (q, J = 7 Hz, 1H: -CHCH₃) ; 5,18 (s large, 1H : -OH) ; 6,86 (d, J = 7,5 Hz, 2H : -C₆H₄OCH₃(-H 3 et -H 5)] ; 7,15 (d, J = 7,5 Hz, 2H : -C₆H₄OCH₃ (-H 2 et -H 6)] ; 7,68 (s, 1H : -H en 4 du thiazolyl-5) ; 8,82 (s, 1H: -H en 2 du thiazolyl-5).
- le mélange en proportion molaire (75/25) des deux diastéréoisomères d'acétoxy-3 [(méthoxy-4 phényl)-1-(S)]éthyl-1 (thiazolyl-5)-4 azétidinone-2, respectivement forme (3R,4R) et forme (3S,4S) sous forme d'une huile épaisse jaune.
- la N-(thiazolyl-5)méthylidène-[(méthoxy-4)phényl-1 éthylamine]-(S) sous forme d'une huile épaisse jaune.
- le thiazolecarbaldéhyde-5 préparé selon la méthode décrite par A. Dondoni et al., Synthesis., 1987, 998-1001

## Revendications

1. Procédé de préparation d'un β-lactame de formule générale : 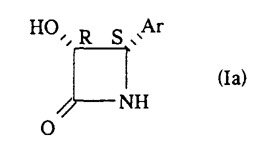 dans laquelle Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles, ou bien Ar représente un radical hétérocyclique aromatique ayant 5 chaînons et contenant un ou plusieurs atomes, identiques ou différents, choisis parmi les atomes d'azote, d'oxygène ou de soufre, éventuellement substitué par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle confient 1 à 4 atomes de carbone ou alcoxycarbonyle dont la partie alcoxy contient 1 à 4 atomes de carbone, caractérisé en ce que
l'on remplace le groupement inducteur chiral d'un produit de formule générale : 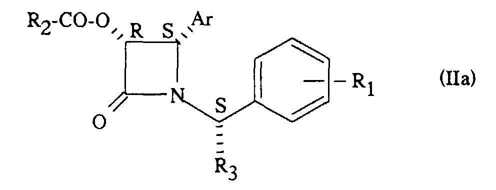 ou d'un mélange de produits de formule générale : 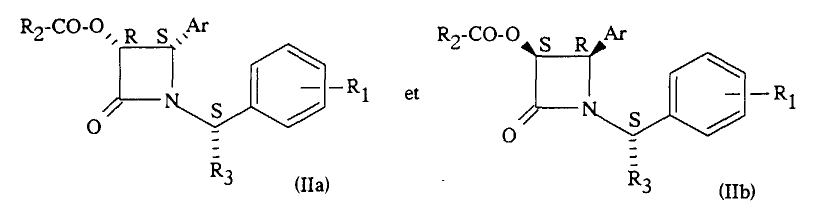 dans laquelle R₁ représente un ou plusieurs substituants dont un doit être en position ortho ou para, identiques ou différents, choisis parmi les radicaux alcoxy contenant 1 à 4 atomes de carbone, alcoylthio dont la partie alcoyle contient 1 à 4 atomes de carbone ou dialcoylamino dont chaque partie alcoyle contient 1 à 4 atomes de carbone, R₂ représente un radical alcoyle contenant 1 à 4 atomes de carbone et R₃ représente un radical alcoyle contenant 1 à 4 atomes de carbone,
pour obtenir le produit de formule générale : 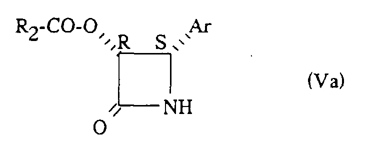 ou un mélange des produits de formule générale : 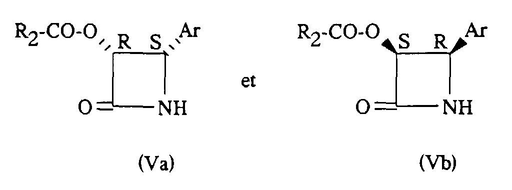 dans laquelle Ar et R₂ sont définis comme précédemment,
que l'on saponifie pour obtenir le produit de formule générale : 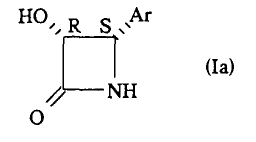 ou le mélange des produits de formule générale : 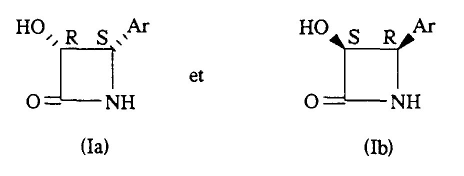 dont on sépare le produit de formule générale (Ia) par cristallisation sélective ou par chromatographie sur phase chirale,
étant entendu que le produit de formule générale (IIa) ou le mélange des produits de formule générale (IIa) et (IIb) est obtenu par action d'un halogénure d'acide de formule générale : 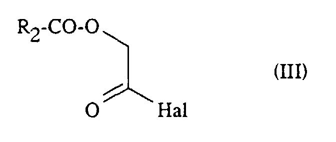 dans laquelle R₂ est défini comme précédemment et Hal représente un atome d'halogène, sur une imine chirale de formule générale : 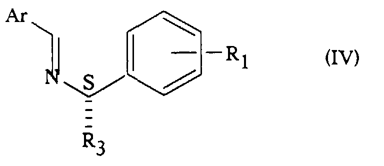 dans laquelle Ar, R₁, R₂ sont définis comme précédemment.

2. Procédé selon la revendication 1 caractérisé en ce que le remplacement du groupement inducteur chiral est effectué par hydrolyse en présence de nitrate de cérium et d'ammonium ou de dichloro-2,3 dicyano-5,6 p-benzoquinone ou d'acétate mercurique ou de bis(trifluoroacétoxy)iodobenzène en opérant dans l'eau ou un milieu hydroorganique à une température comprise entre 0 et 50°C ou par oxydation électrochimique.

3. Procédé selon la revendication 2 caractérisé en ce que le solvant est choisi parmi les nitriles et les esters.

4. Procédé selon la revendication 1 caractérisé en ce que la saponification est effectuée au moyen d'ammoniac en solution dans un alcool aliphatique.

5. Procédé selon la revendication 1 caractérisé en ce que l'on effectue la saponification du mélange des produits de formule générale : 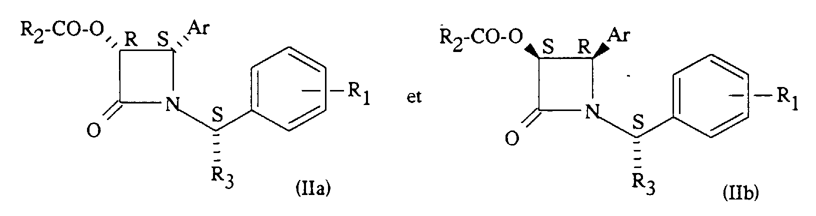 dans lesquelles Ar, R₁, R₂ et R₃ sont définis comme précédemment,
pour obtenir un mélange de produits de formule générale : 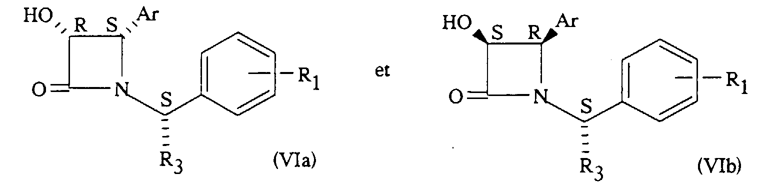 dans lesquelles Ar, R₁ et R₃ sont définis comme précédemment,
dont on sépare le constituant de formule générale (VIa) que l'on estérifie au moyen d'un acide de formule générale :
R₂-CO-OH (VII)
dans laquelle R₂ est défini comme précédemment,
pour obtenir le produit de formule générale : 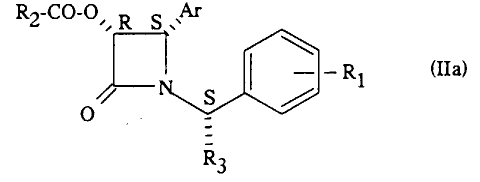 dans laquelle Ar, R₁, R₂ et R₃ sont définis comme précédemment,
dont on remplace le groupement inducteur chiral pour obtenir le produit de formule générale : 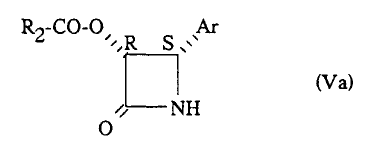 que l'on saponifie pour obtenir le produit de formule générale (Ia),
étant entendu que le mélange des produits de formule générale (IIa) et (IIb) est obtenu par action d'un halogénure d'acide de formule générale : 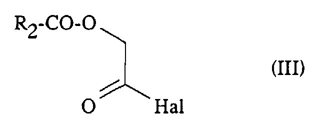 dans laquelle R₂ est défini comme précédemment et Hal représente un atome d'halogène sur une imine chirale de formule générale : 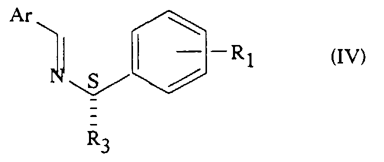 dans laquelle Ar, R₁ et R₃ sont définis comme précédemment.

## Claims

1. Process tor the preparation of a β-lactam of general formula: 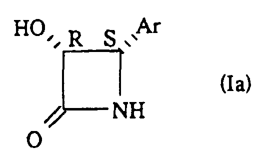 in which Ar represents represents a phenyl or α- or β-naphthyl radical which is optionally substituted with one or more atoms or radicals chosen from halogen atoms (fluorine, chlorine, bromine or iodine) and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alcoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms, that the alkenyl and alkynyl radicals contain 2 to 8 carbon atoms and that the aryl radicals are phenyl or α- or β-naphthyl radicals, or alternatively Ar represents a 5-membered aromatic heterocyclic radical containing one or more atoms, which may be identical or different, chosen from nitrogen, oxygen or sulphur atoms, and which is optionally substituted with one or more substituents, which may be identical or different, chosen from halogen atoms (fluorine, chlorine, bromine or iodine) and alkyl containing 1 to 4 carbon atoms, aryl containing 6 to 10 carbon atoms, alkoxy containing 1 to 4 carbon atoms, aryloxy containing 6 to 10 carbon atoms, amino, alkylamino containing 1 to 4 carbon atoms, dialkylamino in which each alkyl part contains 1 to 4 carbon atoms, acylamino in which the acyl part contains 1 to 4 carbon atoms, alkoxycarbonylamino containing 1 to 4 carbon atoms, acyl containing 1 to 4 carbon atoms, arylcarbonyl in which the aryl part contains 6 to 10 carbon atoms, cyano, carboxyl, carbamoyl, alkylcarbamoyl in which the alkyl part contains 1 to 4 carbon atoms, dialkylcarbamoyl in which each alkyl part contains 1 to 4 carbon atoms, or alkoxycarbonyl radicals in which the alkoxy part contains 1 to 4 carbon atoms, characterized in that the chirality-inducing group of a product of general formula: 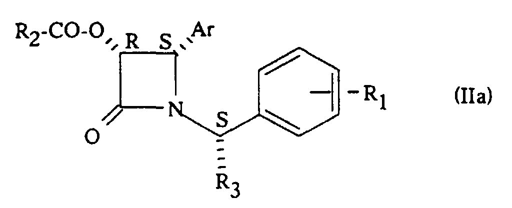 or of a mixture of products of general formulae 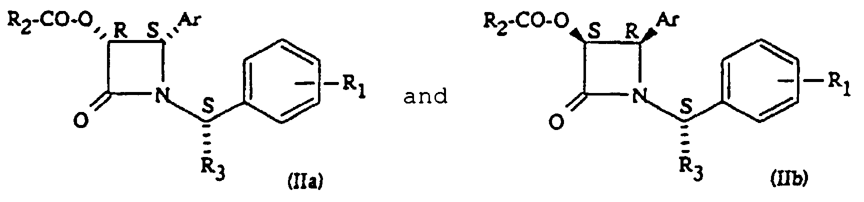 in which R₁ represents one or more substituents of which one must be in the ortho or para position, which are identical or different, and which are chosen from alkoxy containing 1 to 4 carbon atoms, alkylthio in which the alkyl part contains 1 to 4 carbon atoms or dialkylamino radical in which each alkyl part contains 1 to 4 carbon atoms, R₂ represents an alkyl radical containing 1 to 4 carbon atoms and R₃ represents an alkyl radical containing 1 to 4 carbon atoms, is replaced in order to obtain the product of general formula: 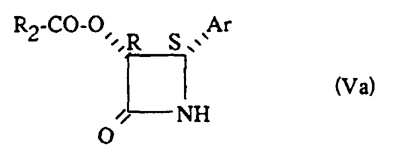 or a mixture of products of general formulae: 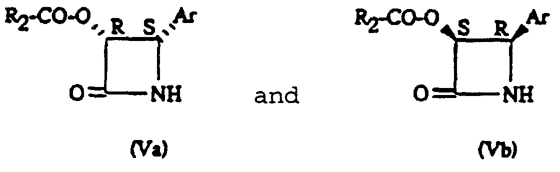 in which Ar and R₂ are defined as above, which product is saponified in order to obtain the product of general formula: 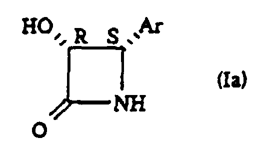 or a mixture of products of general formulae: 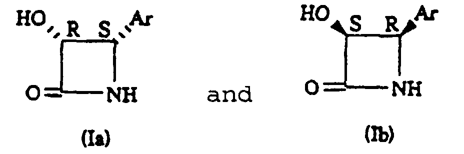 wherein the product of general formula (Ia) is separated out by selective crystallization or by chromatography on a chiral phase,
it being understood that the product of general formula (IIa) or the mixture of products of general formulae (IIa) and (IIb) is obtained by the action of an acid halide of general formula: 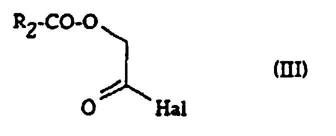 in which R₂ is defined as above and Hal represents a halogen atom, on a chiral imine of general formula: 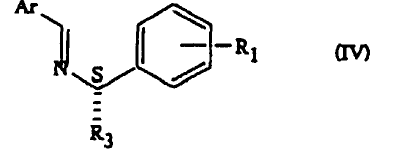 in which Ar, R₁ and R₂ are defined as above.

2. Process according to claim 1, characterized in that replacement of the chirality-inducing group is carried out by hydrolysis in the presence of ammonium cerium nitrate or 2,3-dichloro-5,6-dicyano-p-benzoquinone or mercuric acetate or bis(trifluoroacetoxy)iodobenzene, by working in water or in an aqueous-organic medium at a temperature between 0 and 50°C, or by electrochemical oxidation.

3. Process according to claim 2, characterized in that the solvent is chosen from nitriles and esters.

4. Process according to claim 1, characterized in that the saponification is carried out using ammonia dissolved in an aliphatic alcohol.

5. Process according to claim 1, characterized in that saponification of the mixture of products of general formulae: 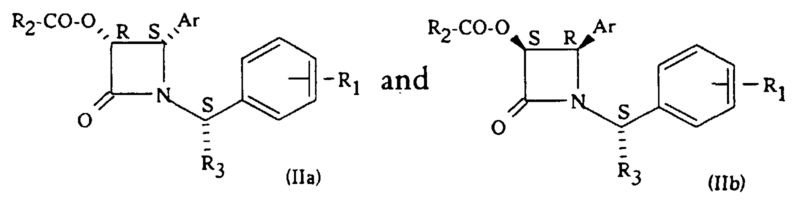 in which Ar, R₁, R₂ and R₃ are defined as above, is carried out
in order to obtain a mixture of products of general formulae: 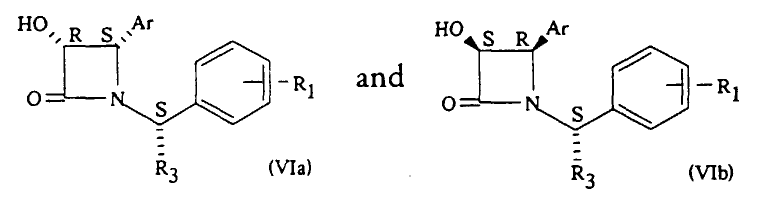 in which Ar, R₁ and R₃ are defined as above, from which the constituent of general formula (VIa) is separated out, which is esterified using an acid of general formula:
R₂-CO-OH (VII)
in which R₂ is detined as above, in order to obtain the product of general formula: 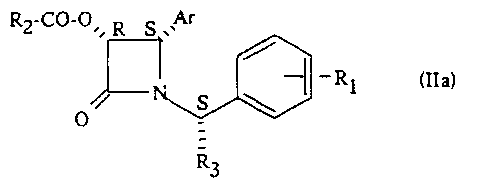 in which Ar, R₁, R₂ and R₃ are defined as above, from which the chirality-inducing group is replaced in order to obtain the product of general formula: 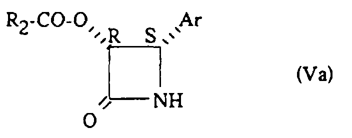 which is saponified in order to obtain the product of general formula (Ia),
it being understood that the mixture of products of general formulae (IIa) and (IIb) is obtained by the action of an acid halide of general formula: 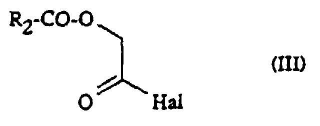 in which R₂ is detined as above and Hal represents a halogen atom, on a chiral imine of general formula: 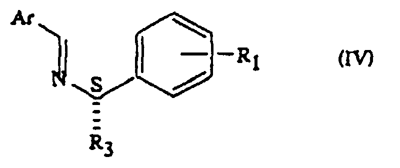 in which Ar, R₁ and R₃ are defined as above.

## Patentansprüche

1. Verfahren zur Herstellung eines β-Lactams der allgemeinen Formel (Ia) 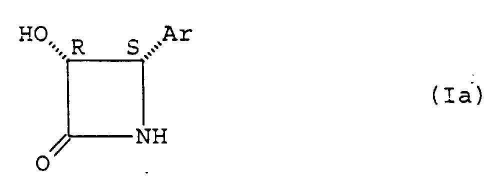 in der
Ar einen Rest Phenyl oder α- oder β-Naphthyl darstellt, gegebenenfalls substituiert durch ein oder mehrere Atome oder Reste, ausgewählt unter den Halogenatomen (Fluor, Chlor, Brom, Iod) und den Resten Alkyl, Alkenyl, Alkinyl, Aryl, Arylalkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Dialkylcarbamoyl, Cyano, Nitro und Trifluormethyl, mit der Maßgabe, daß die Reste Alkyl und die Teile Alkyl der anderen Reste 1 bis 4 Kohlenstoffatome enthalten, daß die Reste Alkenyl und Alkinyl 2 bis 8 Kohlenstoffatome enthalten und daß die Reste Aryl Reste Phenyl oder α- oder β-Naphthyl sind, oder
Ar einen aromatischen heterocyclischen Rest mit 5 Riuggliedern darstellt, enthaltend ein oder mehrere, gleiche oder verschiedene Atome, ausgewählt unter den Atomen von Stickstoff, Sauerstoff oder Schwefel, gegebenenfalls substituiert durch einen oder mehrere, gleiche oder verschiedene Substituenten, ausgewählt unter den Halogenatomen (Fluor, Chlor, Brom, Iod) und den Resten Alkyl mit 1 bis 4 Kohlenstoffatomen, Aryl mit 6 bis 10 Kohlenstoffatomen, Alkoxy mit 1 bis 4 Kohlenstoffatomen, Aryloxy mit 6 bis 10 Kohlenstoffatomen, Amino, Alkylamino mit 1 bis 4 Kohlenstoffatomen, Dialkylamino, bei dem jeder Teil Alkyl 1 bis 4 Kohlenstoffatome enthält, Acylamino, dessen Teil Acyl 1 bis 4 Kohlenstoffatome enthält, Alkoxycarbonylamino mit 1 bis 4 Kohlenstoffatomen, Acyl mit 1 bis 4 Kohlenstoffatomen, Arylcarbonyl, dessen Teil Aryl 6 bis 10 Kohlenstoffatome enthält, Cyano, Carboxy, Carbamoyl, Alkylcarbamoyl, dessen Teil Alkyl 1 bis 4 Kohlenstoffatome enthält, Dialkylcarbamoyl, bei dem jeder Teil Alkyl 1 bis 4 Kohlenstoffatome enthält, oder Alkoxycarbonyl, dessen Teil Alkoxy 1 bis 4 Kohlenstoffatome enthält, dadurch gekennzeichnet, daß man die chirale Induktionsgruppe austauscht bei einem Produkt der allgemeinen Formel (IIa) 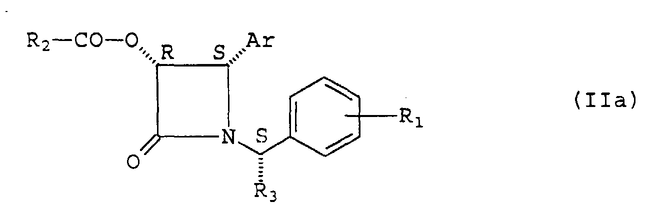 oder bei einer Mischung von Produkten der allgemeinen Formeln 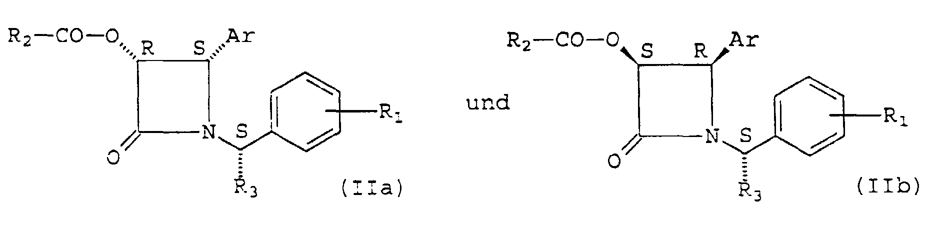 worin R₁ einen oder mehrere, gleiche oder verschiedene Substituenten darstellt, von denen sich einer in Position ortho oder para befinden soll, ausgewählt unter den Resten Alkoxy mit 1 bis 4 Kohlenstoffatomen, Alkylthio, dessen Teil Alkyl 1 bis 4 Kohlenstoffatome enthält, oder Dialkylamino, bei dem jeder Teil Alkyl 1 bis 4 Kohlenstoffatome enthält, R₂ einen Rest Alkyl mit 1 bis 4 Kohlenstoffatomen bedeutet und R₃ ein Rest Alkyl mit 1 bis 4 Kohlenstoffatomen ist, um das Produkt der allgemeinen Formel (Va) 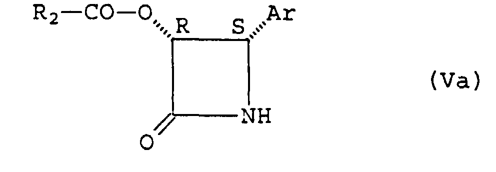 oder eine Mischung von Produkten der allgemeinen Formel 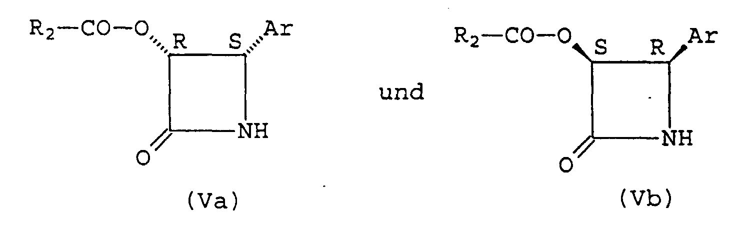 zu erhalten, worin Ar und R₂ wie vorstehend definiert sind, die man verseift, um das Produkt der allgemeinen Formel (Ia) 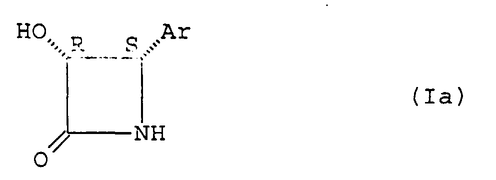 oder die Mischung der Produkte der allgemeinen Formel 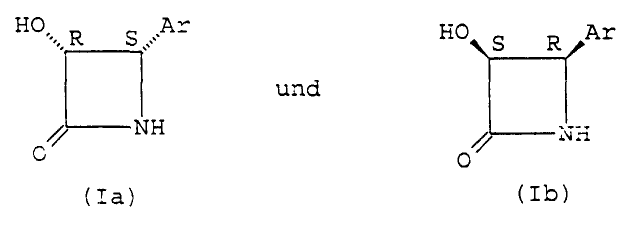 zu erhalten, bei der man das Produkt der allgemeinen Formel (Ia) durch selektive Kristallisation oder durch Chromatographie auf chiraler Phase abtrennt,
mit der Maßgabe, daß das Produkt der allgemeinen Formel (IIa) oder die Mischung der Produkte der allgemeinen Formeln (IIa) und (IIb) durch Einwirkung eines Säurehalogenides der allgemeinen Formel (III) 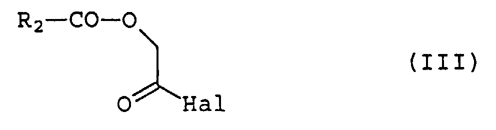 in der R₂ wie vorstehend definiert ist und Hal ein Halogenatom darstellt, auf ein chirales Imin der allgemeinen Formel (IV) 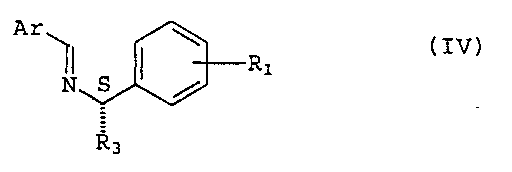 erhalten wird, in der Ar, R₁ und R₃ wie vorstehend definiert sind.

2. Verfahren nach Anspruch 1, dadurch gekennzeichnet, daß der Austausch der chiralen Induktionsgruppe durch Hydrolyse in Anwesenheit von Ammoniumcernitrat oder 2,3-Dichlor-5,6-dicyano-para-benzochinon oder Quecksilberacetat oder Bis-(Trifluoracetoxy)-iodbenzol, wobei man in Wasser oder in einem wäßrig-organischen Medium bei einer Temperatur zwischen 0 °C und 50 °C arbeitet, oder durch elektrochemische Oxidation durchgeführt wird.

3. Verfahren nach Anspruch 2, dadurch gekennzeichnet, daß das Lösungsmittel unter den Nitrilen und den Estern ausgewählt wird.

4. Verfahren nach Anspruch 1, dadurch gekennzeichnet, daS die Verseifung mit Hilfe von Ammoniak in Lösung eines aliphatischen Alkohols durchgeführt wird.

5. Verfahren nach Anspruch 1, dadurch gekennzeichnet, daß man die Verseifung der Mischung der Produkte der allgemeinen Formeln (IIa) und (IIb) 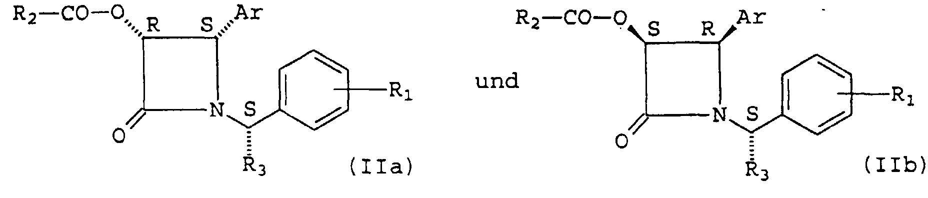 worin Ar, R₁, R₂ und R₃ wie vorstehend definiert sind, durchführt, um eine Mischung von Produkten der allgemeinen Formeln (VIa) und (VIb) 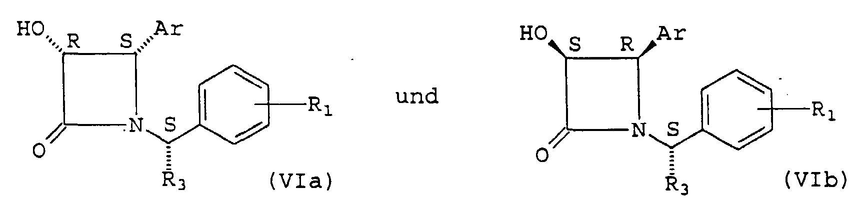 zu erhalten, worin Ar, R₁ und R₃ wie vorstehend definiert sind, bei der man den Bestandteil der allgemeinen Formel (VIa) abtrennt, den man anschließend mit Hilfe einer Formel der allgemeinen Formel (VII)
R₂-CO-OH (VII)
verestert, in der R₂ wie vorstehend definiert ist, um das Produkt der allgemeinen Formel (IIa) 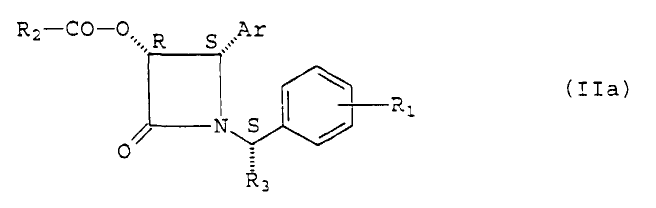 zu erhalten, in der Ar, R₁, R₂ und R₃ wie vorstehend definiert sind, bei dem man die chirale Induktionsgruppe austauscht, um das Produkt der allgemeinen Formel (Va) 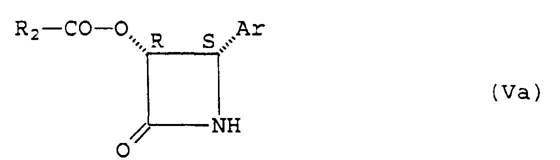 zu erhalten, das man anschließend verseift, um das Produkt der allgemeinen Formel (Ia) zu erhalten,
mit der Maßgabe, daß die Mischung der Produkte der allgemeinen Formeln (IIa) und (IIb) durch Einwirkung eines Säurehalogenides der allgemeinen Formel (III) 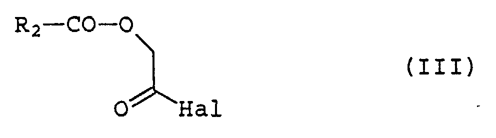 in der R₂ wie vorstehend definiert ist und Hal ein Halogenatom darstellt, auf ein chirales Imin der allgemeinen Formel (IV) 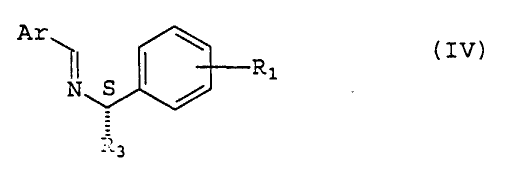 erhalten wird, in der Ar, R₁ und R₃ wie vorstehend definiert sind.
